# EUROPEAN PATENT SPECIFICATION

(11) **EP 1 618 509 B1**
(45) Date of publication and mention of the grant of the patent: **18.04.2012**
(21) Application number: 03721272.7
(22) Date of filing: 29.04.2003
(51) Int. Cl.: G06F 19/00

(54) **SEGMENTAL CODING METHOD AND APPARATUS**
VERFAHREN UND GERÄT ZUR SEGMENTBASIERTEN KODIERUNG
PROCEDE ET DISPOSITIF DE CODAGE SEGMENTAL

(43) Date of publication of application: 25.01.2006
(73) Proprietor: Konuralp, Cüneyt, 34740 Istanbul (TR)
(72) Inventor: Konuralp, Cüneyt, 34740 Istanbul (TR)
(74) Representative: Beresford, Keith Denis Lewis
(86) International application number: PCT/TR2003/000034
(87) International publication number: WO 2004/097709

(56) References cited:
- WO-A-01/11548
- WO-A-02/080108
- US-A- 2 399 424
- E.L. ALDERMAN, K.K. HAMILTON, J. SILVERMAN, D.C. HARRISON, W.J. SANDERS: "Anatomically Flexible, Computer-Assisted Reporting System for Coronary Angigraphy" THE AMERICAN JOURNAL OF CARDIOLOGY, vol. 49, no. 5, 1 April 1982 (1982-04-01), pages 1208-1215, XP008025385 cited in the application
- G. BOZZI, F. CASOLO, M. FERRANTE, F. LO PRESTI, P. TERRANOVA: "Reporting, filing and retrieval of coronary angiographic findings: A new personal computer-based system" CATHETERIZATION AND CARDIOVASCULAR DIAGNOSIS, vol. 13, no. 5, 1987, pages 337-343, XP008025389 cited in the application
- VAN CLEYNENBREUGEL J ET AL: "Annotating radiological images for computer assisted communication and teaching" COMPUTER COMMUNICATIONS, ELSEVIER SCIENCE PUBLISHERS BV, AMSTERDAM, NL, vol. 19, no. 6, 1 June 1996 (1996-06-01), pages 498-508, XP004052736 ISSN: 0140-3664
- XP000747040

## Description

### Technical field

This invention relates to computer apparatus incorporating a program for implementing a novel coding and reporting method, which is capable to describe and present any condition and/or any information embedded in a coronary angiogram in a compact form, with all its properties including its precise coordinates.

### Prior art

Most of the current medical such as cineangiography, radiologic examination (with/without contrast media) of body systems, pathologic examination, echocardiogaphy, etc., are based on the description of visible lesions and anatomy by narrative techniques. The physician examines and interprets the test, for instance cineangiography, and writes down his/her description and commends in the form of a report. Sometimes, instead of or in addition to the written report, the clinician marks several points on freehand or predrawn, standard diagrams.

However, these narrative reports cause a number of difficulties for both the clinician and the patient. First of all, those who read the report may, in general, not be able to understand the exact location of the problematic area. To illustrate, a report sentence like "There is a 60% stenosis in the mid portion of the right coronary artery" may be understood quite differently by everyone to read it. It may be proposed as a solution to give more details in the report text, but this will eventually result in a lengthy and unintelligible report, which will, in turn, render difficult to draw precise and quick inferences. Furthermore, freehand diagrams to accompany and increase the legibility of these reports do not have the desired reproducibility and teachability, whereas standard diagrams are not flexible enough to accommodate variations in coronary anatomy which differ extensively from patient to patient. As a result, with or without a diagram, these reports generally do not supply enough practical information to the clinicians.

In addition to the above-mentioned problems, current narrative reporting systems have no uniformity and differ from one country to another and one institution to the other The fact that these reports are written in the official languages of the countries where they are arranged, restricts their validity within the territories of those countries.

Another difficulty arises especially for the cases involving more than one pathologies in close vicinity to each other. It is not possible to numerate or sign these pathologies in the classical system. Therefore, the clinician to refer to only a selected one of these pathologies, has to first describe its place, which is tedious. Even if s/he is succesful to describe it somehow, everyone might not understand the same pathology from his/her report. Moreover, sequential angiograms for the same patent cannot be compared easily as these do generally not stand on a common base.

Finally, such narrative reports can be stored in computers only by word-processing programs, which are not quite purpose fit for data processing. Thus, statistical studies become even more tedious.

Various solutions have been proposed in the prior art, to overcome the above-mentioned problems arising from narrative reporting methods. Brandt et aL (Coronary arteriography; method of presentation of the arteriogram report and a scoring system. Clinical Radiology. 1977; 28(4):361-5.) proposed a method named as Green Lane system. This system is based on presentation on a special drawn diagram. Right and left ventricles, and inter ventricular septum are drawn schematically and divided into nine segments. Coronary arteries are schematized according to their properties to perfuse these areas and abnormal findings such as aneurysm, etc. are marked on this according to some predetermined rules.

Furthermore, a new pointing system named as total jeopardized score is introduced as a new criterion for revascularisation indication. A modified Green Lane system is developed by P. K. Goel (A relook at the reporting of coronary angiograms in the interventional era: A perspective. Indian Heart Journal 1997; 49(3):323-6.) which allows more information to be presented but does not involve the use of jeopardized score. However, both systems are based on a fictitious diagram which does not help the interventional cardiologist or the surgeon to view the real anatomy of the patient.

The method proposed by Bozzi et al. (Reporting, filing, and retrieval of coronary angiographic findings: A new personal computer-based system. Catheterization and Cardiovascular Diagnosis 1987; 13(5):337-43.) comprises the step of answering every multiple-choice question of a computer program which in turn forms a diagram accordingly. The closed-end character of multiple-choice questions renders this system insufficient for the presentation of special cases.

Alderman et al. developed another method (Anatomically flexible computer-assisted reporting system for coronary angiography. American Journal of Cardiology 1982; 49(5): 1208-15.) which utilizes a touch-matic computer screen. There is a template illustrating a normal coronary artery anatomy. The reporter uses the touch-matic screen to mark various points and/or modifications on this anatomy.

Budkin et al (On-line computer storage, retrieval, and reporting of coded angiographic data. Radiology. 1978; 127(1):141-5.) describe a method which aims at standardization in reporting. Among various situations presented by the computer, the most suitable ones are selected and a written text is amended if necessary. Melendez et al introduced a similar system (Low-cost comprehensive data system for cardiac patients. Catheterization and Cardiovascular Diagnosis 1979; 5(4):347-55.) incorporating a printed form. Sapoznikov et al proposed (A graphic computerized system for reporting and analysis of coronary angiographs.

Computers and Biomedical Research 1983; 16(4):334-9.) a system which involves three different variants of the arterial anatomy in a computer. The coronary artery tree of each variant is divided into more than a hundred segments and each segment is numbered accordingly. The reporter determines the problematic area in the arteriogram, matches a segment number to this area from the diagram, and writes down his findings. The computer presents the findings on the relevant segments graphically. Piessens and Willems (A computer based coronary arteriogram reporting and information system. Acta Cardiologica Supplementum 1981; 26:59-67.) developed a similar method which makes use of pre-determined segments for the artery system and codes to be entered in a form. Basically, all of the methods mentioned above are based on standard diagrams; hence they are not capable of communicating the most precise information in the most understandable form

In prior art document WO 02/080108 Al, a device, method and a computer program to model and report an anatomic structure, including a coronary angiography is provided. The user starts from templates describing standard morphologies, thus this invention also suffers from the weaknesses of previously descried methods/systems.

### Object of the Invention

The object of the present invention is to provide a computer apparatus which implements a segmental coding and reporting method based on a novel mapping method and which is capable to compactly describe any condition and/or any information embedded in a related coronary angiogram with all necessary properties including its exact coordinates, and that is globally understandable and fully suitable for computerization and statistical retrieval.

The invention is defined in claim 1.

### Explanation of the Invention

The method subject to this invention is best described by the accompanying drawings attached hereto. The drawings are explained herebelow:
Figure 1- An exemplary map.
Figure 2- Exemplary mapping of coronary artery.
Figure 3- An exemplary coronary angiogram.

The parts in the drawings are individually enumerated and explained hereafter:
1. Map
2. Marker point
3. Segment unit
4. Stent
5. Aneurysm
6. Thrombus

The present invention is based on a unique mapping, coding and formulation method.

The mapping, depends on the neighbourhood relationship between anatomic parts. Even though anatomy may change considerably from one patient to another, the neighbourhood relation will remain constant. Hence, mapping and addressing a lesion or an anomaly, based on this principle is capable of presenting any kind of anatomical variation. This map (1) is composed of marker points (2), and segment units (3) denoting a part of the anatomy in relation to marker points (2).

Determination of the marker points (2) is important for the efficiency and coherency of the method.

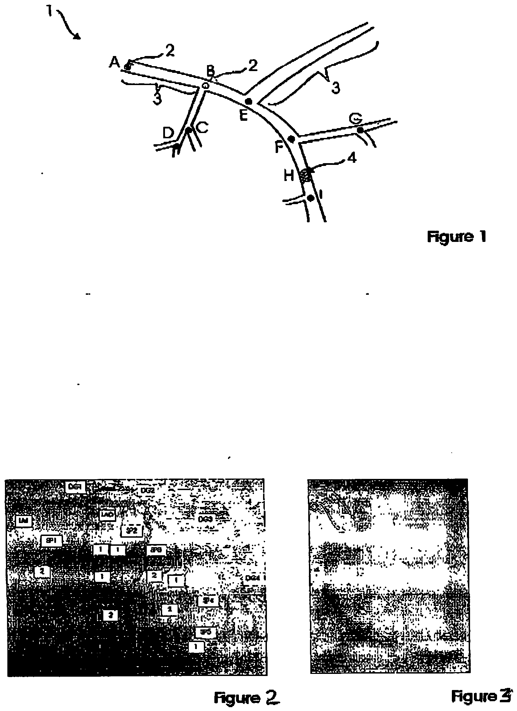

The marker points (2) of a coronary artery are branching points, junctions of the vessels (Figure 1). In cases where necessary, benchmark points of the anatomy, be them genuine or artificial, such as locations of proximal and distal anastomoses of the bypassed grafts, Intraluminally placed stents (4) may also be utilized as marker points (2) (Figure 1). If no marker points (2) could be found, this is mentioned by for example an asterisk sign (*) in the coordinate field, with an aim to warn the reader. In such a case, the segment as seen on the diagram is divided preferably into three or more segments and segments are referred to by their closeness, such as proximal, mid and distal, etc. The segment units (3) are lines between two marker points (2) and/or originating from a marker point (2) (Figure 1). Thus the segment units (3) of the coronary artery (Figure 1), are lines coinciding with vessel segments extending between two marker points (2). It is of uppermost importance that the segments are defined between marker points (2) lying on vessels at the same level. In other words, a segment unit (3) should not start from a marker point (2) on a primary branch and terminate at a marker point (2) on a secondary or tertiary branch. The shorter the segment unit (3) is defined, the more precise the coordinates are given.

Marker points (2) and segment units (3) can be viewed as corners and streets in a city map, respectively. In order to reduce the language dependency of the map (1) and thereby the report, marker points (2) and segment units (3) are coded using previously determined letters, numerals, symbols and/or signs.

Once the mapping is established, any anatomical condition can be localized using the predetermined codes on the map. The report is presented in the form of a plurality of independent formulae, each consisting of a plurality of different information fields separated by symbols, preferably colons (:) or equal signs (=). The formula for each condition has the following format:
Condition n: Subject: Information [Supportive data]

In the above formula, "condition" in the first field refers to any situation, e.g. pathology regarding a region. If we are to exemplify, conditions for coronary artery could be stenosis, occlusion, contour deformity, aneurysm (5), dissection, rupture, existence of stent, gradient, visualisation etc. Still for the aim of shortening the formulae and making it language-independent, conditions are coded using alphanumeric characters and/or symbols. The reporter may create new codes, if s/he finds necessary. In one embodiment of the invention, the condition field may involve more than one conditions related to each other, separated by symbols/signs, preferably colons (:).

"n" is the identification number of the condition. This is basically a sequence number given to each condition of the report starting from 1. For instance, if there are, say, a total of 12 stenoses in a report they are coded and enumerated from S1 to S12 Once this identification number is given to a condition in a report, it becomes permanent and this condition is referred to by this number in the later reports. Identification number is preferably in Arabic numerals; however it may be in Roman numerals as well.

Subject is simply the site to which the condition is related, namely the vessel, cardiac chamber, valve, etc. Similar to other fields, these sites are inserted in the formula with codes.

"Information," as the name implies, is the data about the subject and/or the condition. This data may contain coordinates and/or scores. Coordinates are the spatial and/or temporal information of the condition with regards to the map. Score is any alphanumerical value regarding the condition. If both coordinate and score information is to be given in the formula, they are separated by colons. Coordinates identify the exact spatial and temporal location and/or direction of the condition on the condition or subject. These coordinates are given according to the mapping i.e. the marker points (2) and segment units (3) formed previously. It is an important point that coordinates are employed to denote the time dimension, as well as the spatial dimension. One application of time dimension is with cineangiograms, where sequential frames of the film are referred to by time coordinates. Moreover, key symbols are provided within the coordinate information to be able to provide the report with other spatial/temporal information such as), distal 1/3, proximal 1/3, middle 1/3, before, after, junction, etc. If a lesion is to be descried in the coordinate field and if this lesion is less than nearly 0.5 cm, then the lesion is deemed discrete and only center coordinates of this lesion is given in the formula. On the contrary, if the lesion is equal or longer than 0.5 cm, it is deemed segmental, and coordinates of both end points of the lesion are given.

Score is the term which provides alphanumerical information about the condition, if necessary. For some conditions, such as stenoses, stent, etc. only coordinate information may suffice. However, for some other conditions like hemodynamic measurement an alphanumerical value, that's to say a score must be provided. These scores may involve, grades of ventricle wall segment motion, degree of valve insufficiency, blood/gas pressure, saturation value, risk score, eco score, etc.

Finally, additional information regarding the condition is placed in the "supportive data" field, preferably within square brackets. This field may be used to give necessary information which cannot be placed in any of the previous fields. This feature increases the flexibility of the method to cope with any unforeseen situations and interactivity of the method with the reporter.

Once the formulation is completed, the report is prepared by writing necessary formulae one after the other. Formulae may be placed in the same line or preferably, in following lines.

In this invention, an apparatus for realizing various steps of this method is utilized. This apparatus may be a computer terminal, which incorporates a program according to an algorithm for easing mapping, storing predetermined code tables for different fields of the formula, inputting information, creating formula, forming the final report, storing said report, outputting it, drawing of customized diagram from the formulae, and searching the database among a plurality of reports.

As it will be difficult for ordinary people and the physicians who are not familiar to the format, say patient/physician, to understand such coded and compact reports, said apparatus may further comprise means for converting formula into an extended form. This extended form may have two different embodiments: In the first embodiment, the codes in the formula will be matched from the tables of codes and will be presented in the standard format of the formula, but in a decoded form. In a second embodiment, codes in the formula will be converted to complete sentences, and the report will be in a narrative format.

In still another embodiment, the apparatus may further involve means for converting the formula to extended form in any selected language. This apparatus may further include means to present various codes in one formula of the report with link to other formulae involving this same code.

In another embodiment, the apparatus also involves means for running attached sound (such as waw, mp3) and image files related to the subject of the report. The image files may be in any of the known formats such as jpg, pdf, tif, or streaming image formats such as mva, mpeg. Exemplary situations of where video files may be needed are movies (MOVn) or injections (ENJn).

### Examples

First, a coronary angiogram is mapped according to the mapping principles discussed above (Figure 2). In this map, primary, secondary, tertiary, quartery, etc. branches are named accordingly. Then various conditions of this angiogram is formulated using the codes given in Tables 1-4 below, and according to the rules of the formulation mentioned above. Some formulae and their meanings, in other words their extended form, are provided below.

### VIS3: RCA: S [A1R3 LAD]

Visualization number three. Right coronary artery. It seems to be thin (less then 1.5-mm in-diameter). According to TIMI grading, antegrade filling score is 1 and retrograde filling is 3 and this happens via collaterals originating from LAD.

### S4: LAD: b DG1- a DG1 [40%, seg, 2.5 cm]

Stenosis number 4. On LAD. There is a 40%-, 2.5-cm segmental stenosis, which starts immediately before and terminates immediately after branching to the first diagonal.

### S8: LAD DG2: a 1- * (p) [60%, seg, 3 cm]

Stenosis number 8. On the second diagonal branch of the LAD. 60%-, 3-cm-length, segmantal stenosis. It starts at a point after giving its 1st branch. No anatomic marker (tertiary branch) is seen to refer to the termination point of the stenosis. However, if the portion from the first branching point of the 2nd diagonal artery to its last point that can be seen on the angiogram is accepted as a segment, then stenosis is terminated on the proximal one-third portion of this segment.

### S9: DG2: j DAN2 [40%, cs]

### DAN2: SVI-DG2b I

In the formulae above, DAN2 code in the former is linked to DAN2 in the latter formula. When DAN2 in the former formula is for instance clicked by a mouse, either the latter formula appears on the screen or a small message window that contains the description of DAN2 appears when a mouse arrow is holding in the linked word.

As another example, the two pathologies in Figure 3, namely an aneurysm (5) and a thrombus (6), are formulated in the report as follows:
ANR1: RCA: a SNA-b RVA
THR1: RCA: b RVA

**Table-1: The list of condition codes**

| **Condition n** | |
|---|---|
| **n** | Identification number of the condition (1, 2, 3, ...) |
| **DOM** | Dominance |
| **S** | Stenosis |
| **SPS** | Spasm |
| **REC** | Recanalization |
| **CD** | Contour deformity (diffuse disease) |
| **CAL** | Calcification |
| **ANR** | Aneurysm |
| **DS** | Dissection |
| **RT** | Rupture |
| **BRG** | Bridge lesion |
| **ST** | Existence of stent |
| **AV** | Anatomic variation |
| **OA** | Origin anomaly |
| **DA** | Distribution (routing) anomaly |
| **TA** | Termination anomaly |
| **PAN** | Proximal anastomosis |
| **DAN** | Distal anastomosis |
| **UND** | Undefined lesion |
| **THR** | Thrombus |
| **WMS** | Wall motion status |
| **REG** | Regurgitation (grade: 1-4) |
| **GRD** | Gradient |
| **VIS** | Visualization |
| **ENJ** | Enjection |
| **MOV** | Movie |
| **COR** | Correction |
| **PRS** | Pressure |
| **SAT** | Saturation |
| **HEM** | Hemodynamics |

**Table-2: The list of angiographic segment and anatomic marker points.**

| **Location Codes** | | |
|---|---|---|
| **ROS** | Right coronary ostium | |
| **LOS** | Left coronary ostium | Coronary ostia |
| **SCO** | Single coronary ostium | |
| **LM** | Left main coronary artery | |
| **LAD** | Left anterior descending coronary artery | |
| **CX** | Circumflex coronary artery | Primary branches |
| **RCA** | Right coronary artery | |
| **IM** | Intermedier coronary artery | |
| **DGn** | Diagonal branch(es) | |
| **SPn** | Septal branch(es) | |
| **OMn** | Obtuse margin branch(es) | |
| **PD** | Posterior descending branch | |
| **PL** | Posterolateral branch | |
| **AM** | Acute margin branch | Secondary branches |
| **COA** | Conus artery | |
| **RAA** | Right atrial branch | |
| **RVA** | Right ventricular branch | |
| **SNA** | Sinus node artery | |
| **AVN** | Atrioventricular node artery | |
| **1, 2, 3, ...** | Subordinate(s) | Tertiary, quartery branches |
| **SVn** | Saphenous vein | |
| **RIMA** | Right internal mammarial artery | |
| **LIMA** | Left internal mammarial artery | |
| **FIMAn** | Free internal mammarial artery | Grefts |
| **GEA** | Gastroepiploic artery | |
| **FGEA** | Free gastoepiploic artery | |
| **RDn** | Radial artery | |
| **RA** | Right atrium | |
| **RV** | Right ventricle | |
| **LA** | Left atrium | |
| **LV** | Left ventricle | |
| **AO** | Aorta | |
| **VCS** | Vena cava superior | |
| **VCI** | Vena cava inferior | |
| **MPA** | Main pulmonary artery | |
| **RPA** | Right pulmonary artery | |
| **LPA** | Left pulmonary artery | |
| **CS** | Coronary sinus | Extracoronary chambers |
| **RSV** | Right sinus valsalva | |
| **LSV** | Left sinus valsalva | |
| **NCS** | Non coronary sinus | |
| **RVOT** | Right ventricular outflow tract | |
| **LVOT** | Left ventricular outflow tract | |
| **AOV** | Aortic valve | |
| **MV** | Mitral valve | |
| **TV** | Tricuspid valve | |
| **PV** | Pulmonary valve | |

| | | |
|---|---|---|
| **O:** The origin (ostium) of the artery | | |

**Table-3: List of key symbols**

| **Key Symbols** | |
|---|---|
| **:** | Description key |
| **.** | Mode (separator for the levels) |
| **-** | Between |
| **b** | Before |
| **j** | Junction |
| **a** | After |
| **ant** | Anterior |
| **pos** | Posterior |
| ***** | The site has no any anatomic marker |
| **(p)** | Proximal 1/3 portion of the segment |
| **(m)** | Mid 1/3 portion of the segment |
| **(d)** | Distal 1/3 portion of the segment |
| **(p-m)** | Between proximal 1/3 and mid 1/3 portion of the segment |
| **(m-d)** | Between mid 1/3 and distal 1/3 portion of the segment |
| **[cs]** | Consentric |
| **[ec]** | Eccentric |
| **[seg]** | Segmental (tubular) (≥ 0.5 cm-length) |
| **[ulc]** | Ulcerative |
| **[seq]** | Sequential |
| **[ngI]** | Nitroglycerine enjection |
| **@** | At |
| **X** | Crossing |
| **←** | Originating from |
| **→** | Terminating to |
| **A₀₋₃** | Antegrade filling (TIMI 0,1, 2, 3)^{##} |
| **R₀₋₃** | Retrograde filling (TIMI 0, 1, 2, 3)^{##} |
| **L** | Large (≥ 2.0 mm in diameter) |
| **M** | Medium (1.5-2.0 mm in diameter) |
| **S** | Small (< 1.5 mm in diameter) |
| **P** | Poorly visualized |
| **AB** | Absent |
| **NV** | Not visualized |
| **{R}** | Right dominance |
| **{L}** | Left dominance |
| **{B}** | Balanced (mixed) dominance |

| | |
|---|---|
| ^{#}: []: Includes key statements providing extra information on the condition. ^{##}: TIMI (Thrombolysis In Myocardial Infarction) flow grading system: ***Grade 0:*** No flow in the occlusion, ***Grade 1:*** Contrast goes beyond occlusion but fails to opacify the entire bed ***Grade 2:*** Entire vessel opacified, but the rate of entry to and clearance from the distal bed of the contrast is slower than normal ***Grade 3:*** Normal flow | |

**Table-4: Left ventricle wall motion situation (WMS) analysis**

| **Segment Codes** | | **Grade (0-5)** | |
|---|---|---|---|
| **AB** | Anterobasal | | |
| **AL** | Anterolateral | **0** | Normal |
| **AP** | Apical | **1** | Mildly hypokinetic |
| **DP** | Diaphragmatic | **2** | Moderately hypokinetic |
| **PB** | Posterobasal | **3** | Severely hypokinetic |
| **PL** | Posterolateral | **4** | Akinetic |
| **SP** | Septal | **5** | Dyskinetic |
| **GL** | Global | **0-3** (overall left ventricular function) | |

## Claims

1. Computer apparatus for implementing a method of coding conditions and information embedded in a coronary angiogram and incorporating a program according to an algorithm for easing mapping, storing predetermined code tables for different fields of a formula, inputting information, creating said formulae, forming a final report, storing the report, outputting said report, drawing a customised diagram from said formulae, and searching a database having a plurality of said reports; wherein
(a) a first of said tables (table 1) stores a plurality of condition codes and, in association with each of said condition codes, the name of the pathological condition to which the respective code relates;
(b) a second of said tables (table 2) stores a plurality of location codes and, in association with each of said location codes, the name of the location to which the respective location code relates, said locations being
(i) the ostia and the primary, secondary, tertiary and quaternary branches therefrom,
(ii) grafts, and
(iii) extracoronary chambers;
(c) a third of said tables (table 3) stores key symbols for defining spatial positions and, in association with said key symbols, the meanings of the respective key symbols; and
(d) said fields of each formula comprise
(i) a first field which contains a said condition code to identify the condition to which the formula relates and a sequence number for said condition,
(ii) a second field which contains a said location code to identify the site of the condition identified in the first field, and
(iii) a third field which contains appropriate ones of said location codes and said key symbols for defining the spatial position of said condition within the site identified by the location code in the second field, the location codes and key symbols in the third field being such that said spatial position within the site identified in the second field is defined as being before, after or between marker points constituted by junctions of the site identified in the second field with other sites.

## Patentansprüche

1. Computervorrichtung zur Implementierung eines Verfahrens zum Codieren von Zuständen und Informationen, die in einem koronaren Angiogramm eingebettet sind, und mit einem Programm entsprechend einem Algorithmus, um ein Abbilden, Speichern vorbestimmter Code-Tabellen für verschiedene Informationsfelder, Eingeben von Informationen, Erzeugen von Formeln, Bilden eines Abschlussberichts, Speichern des Berichts, Ausgeben des Berichts, Aufstellen eines angepassten Diagramms aus den Formeln und Durchsuchen einer Datenbank mit mehreren solcher Berichte zu unterstützen, wobei
(a) eine erste der Tabellen (Tabelle 1) mehrere Zustandscodes und jedem der Zustandscodes zugeordnet den Namen des pathologischen Zustands, auf den sich der entsprechende Code bezieht, speichert,
(b) eine zweite der Tabellen (Tabelle 2) mehrere Ortscodes und jedem der Ortscodes zugeordnet den Namen des Orts, auf den sich der entsprechende Ortscode bezieht, speichert, wobei die Orte
(i) die Ostien und deren primäre, sekundäre, tertiäre und quartäre Abzweigungen,
(ii) Implantate und
(iii) extrakoronare Kammern sind,
(c) eine dritte der Tabellen (Tabelle 3) Schlüsselsymbole zur Festlegung räumlicher Lagen und den Schlüsselsymbolen zugeordnet die Bedeutungen der entsprechenden Schlüsselsymbole speichert, und
(d) die Felder der jeweiligen Formel folgendes umfassen:
(i) ein erstes Feld, das einen solchen Zustandscode zur Identifizierung des Zustands, auf den sich die Formel bezieht, und eine Abfolgenummer für den Zustand enthält,
(ii) ein zweites Feld, das einen solchen Ortscode zur Identifizierung des Orts des im ersten Feld identifizierten Zustands enthält, und
(iii) ein drittes Feld, das in geeigneter Weise welche der Ortscodes und der Schlüsselsymbole zur Festlegung der räumlichen Lage des Zustands innerhalb des vom Ortscode im zweiten Feld identifizierten Orts enthält, wobei die Ortscodes und Schlüsselsymbole im dritten Feld so eingerichtet sind, dass die räumliche Lage innerhalb des im zweiten Feld identifizierten Orts als vor, nach oder zwischen Markierungspunkten festgelegt ist, die durch Verbindungen des im zweiten Feld identifizierten Orts mit anderen Orten gebildet sind.

## Revendications

1. Appareil informatique permettant d'exécuter un procédé de codage d'états et d'informations intégrés dans un angiogramme coronaire, et d'incorporer un programme selon un algorithme pour faciliter le fait de mapper, de stocker des tables de code prédéterminées pour différents champs d'une formule, le fait d'introduire des informations, de créer lesdites formules, de former un rapport final, de stocker le rapport, de délivrer ledit rapport, de dessiner un diagramme personnalisé à partir desdites formules, et de rechercher dans une base de données comprenant une pluralité desdits rapports ; où
(a) une première table desdites tables (table 1) stocke une pluralité de codes d'états et, en association avec chacun desdits codes d'état, le nom de l'état pathologique auquel le code respectif se rapporte ;
(b) une deuxième table desdites tables (table 2) stocke une pluralité de codes d'emplacement et, en association avec chacun desdits codes d'emplacement, le nom de l'emplacement auquel le code d'emplacement respectif se rapporte, lesdits emplacements étant
(i) les ostiums et les branches primaire, secondaire, tertiaire et quaternaire s'étendant de ceux-ci,
(ii) des greffes, et
(iii) des chambres extracoronaires ;
(c) une troisième table desdites tables (table 3) stocke des symboles clés pour définir des positions spatiales et, en association avec lesdits symboles clés, les significations des symboles clés respectifs ; et
(d) lesdits champs de chaque formule comprennent
(i) un premier champ qui contient ledit code d'état pour identifier l'état auquel la formule se rapporte et un numéro de séquence pour ledit état,
(ii) un deuxième champ qui contient ledit code d'emplacement pour identifier le site de l'état identifié dans le premier champ, et
(iii) un troisième champ qui contient des codes et des symboles appropriés desdits codes d'emplacement et desdits symboles clés pour définir la position spatiale dudit état dans le site identifié par le code d'emplacement dans le deuxième champ, les codes d'emplacement et les symboles clés dans le troisième champ étant tels que ladite position spatiale dans le site identifié dans le deuxième champ est définie comme étant avant, après ou entre des points de marquage constitués en reliant le site identifié dans le deuxième champ aux autres sites.
